# EUROPEAN PATENT APPLICATION

(11) **EP 3 822 370 A1**
(43) Date of publication of application: **19.05.2021**
(21) Application number: 19209383.9
(22) Date of filing: 15.11.2019
(51) Int. Cl.: C12Q 1/689

(54) **METHOD OF DETERMINING THE PRESENCE OF A HYPER-VIRULENT CLOSTRIDIOIDES DIFFICILE STRAIN OF THE B1/NAP1/027 GROUP IN A SAMPLE**

(71) Applicant: Curiosity Diagnostics Sp. z o.o., 01-796 Warsaw (PL)
(72) Inventor: Gewartowski, Kamil, 02-127 Warsaw (PL); Baranowski, Bartosz, 05-800 Pruszków (PL); Kozlowicz, Anna, 01-910 Warsaw (PL)
(74) Representative: Prinz & Partner mbB

(57) **Abstract**

The present invention relates to primer pair and an oligonucleotide probe for hybridizing to at least a portion of a chloramphenicol acetyltransferase gene coded in a transposon of family CTn-027 of a hyper-virulent *Clostridioides difficile* strain of the B1/NAP1/027 group, an oligonucleotide set for use in a nucleic acid amplification process or a nucleic acid detection process for determining the presence of a hyper-virulent *Clostridioides difficile* strain of the B1/NAP1/027 group in a sample, a kit for detecting the presence of a hyper-virulent strain of *Clostridioides difficile* of the B1/NAP1/027 group in a sample, and a method of determining the presence or absence of a hyper-virulent *Clostridioides difficile* strain of the B1/NAP1/027 group in a sample.

SEQUENCE LISTING

<110> Curiosity Diagnostics Sp. z o.o.

<120> Method of determining the presence of a hyper-virulent Clostridioides
difficile B1/NAP1/027 strain in a sample

<130> C4703EP

<160> 4

<170> BiSSAP 1.3.6

<210> 1
<211> 25
<212> DNA
<213> Artificial Sequence

<220>
<223> NAP1F

<400> 1
gggagaagta taaaacagaa cgtgc                                          25

<210> 2
<211> 21
<212> DNA
<213> Artificial Sequence

<220>
<223> NAP1R

<400> 2
gccccaagta ataactggtg c                                              21

<210> 3
<211> 645
<212> DNA
<213> Clostridium

<220>
<223> chloramphenicol acetyltransferase gene

<400> 3
atgggcatgg aatataaata tacaaaaatt gatttgaata aatggaatag aggtaaattg      60

tttcaaagct acattgataa tatgcgtatt gttatgagct taaccgtaga tattgatgta     120

acaaagttta ttgaattttc aaagaagcat gatttaaaat tttatccagc tatgatatgg     180

gctgtttcaa aggttgttaa tgaacatgat gagttcaaat atagttggga taataatggg     240

aatttaatta agtgggatta tatttctccg tcctatactg aatttcataa agaagatgaa     300

aatttcacaa agatagttac agatttttct gatagcatat ttgaatttca taaacgtttt     360

atattagata gggagaagta taaaacagaa cgtgcatttg tggaaaatca atctttgaac     420

ttttttgatg tatcttgttt accgtgggta aaatataatc attttgatgt tcatgttttt     480

gacgagggaa aatttcttgc accagttatt acttggggca aatatgaatt agagcaagaa     540

agatatataa tgcctcttac aatgaatata catcatgccg tagcagatgg attccattta     600

tccagatttt ttaatgaagt acaagaattg ataaactcaa tttaa                     645

<210> 4
<211> 149
<212> DNA
<213> Clostridium

<220>
<223> Part of Chloramphenicol acetyltransferase gene to which primers
      hybridize

<400> 4
   gggagaagta taaaacagaa cgtgcatttg tggaaaatca atctttgaac ttttttgatg     60
   tatcttgttt accgtgggta aaatataatc attttgatgt tcatgttttt gacgagggaa    120
   aatttcttgc accagttatt acttggggc                                      149

## Description

### TECHNICAL FIELD:

The present invention relates to primer pair and an oligonucleotide probe for hybridizing to at least a portion of a chloramphenicol acetyltransferase gene coded in a transposon of family CTn-027 of a hyper-virulent *Clostridioides difficile* strain of the B1/NAP1/027 group, an oligonucleotide set for use in a nucleic acid amplification process or a nucleic acid detection process for determining the presence of a hyper-virulent *Clostridioides difficile* strain of the B1/NAP1/027 group in a sample, a kit for detecting the presence of a hyper-virulent strain of *Clostridioides difficile* of the B1/NAP1/027 group in a sample, and a method of determining the presence or absence of a hyper-virulent *Clostridioides difficile* strain of the B1/NAP1/027 group in a sample.

### BACKGROUND ART:

*Clostridioides difficile* (synonym "*C*. *difficile",* previously named *Clostridium difficile,* Non-Patent literature 1) is a sporulating Gram-positive bacillus producing a pathogenic toxin mediating intestinal diseases in humans and animals. Dependent on the age of the patient, intestinal flora and the *Clostridioides difficile* strain the clinical outcome of a *Clostridioides difficile* infection (CDI) may be asymptomatic or may range to severe disease syndromes, such as diarrhea, abdominal pain, fever and leukocytosis.

Meanwhile, *Clostridioides difficile* is recognized as a main cause for infectious diarrhea in hospitalized patients or patients undergoing a treatment damaging the normal intestinal flora, such as antibiotic or anticancer treatment. *Clostridioides difficile* may, as a consequence of the damage to the intestinal flora produced by the mentioned treatments overgrow and produce toxin A, toxin B and binary toxin CDT, which develop symptoms, such as diarrhea. *Clostridioides difficile* is excreted in feces from infected individuals, and transfection to other individuals may occur orally, in particular by the mucosa of the mouth by colonized hands or instruments.

Currently, a *Clostridioides difficile* strain known as type B1 by Restriction Endonuclease Analysis, North American (NA) pulsed-field type 1 (NAP1) by Pulsed-field Gel Electrophoresis, and 027 by PCR-ribotyping (B1/NAP1/027) is regarded the single most important epidemic strain causing *Clostridioides difficile-*associated disease in North America and Europe (Non-Patent Literature 2).

Thus, there is a need to determine the presence of hyper-virulent *Clostridioides difficile,* in particular *Clostridioides difficile* B1/NAP1/027 strains.

Presently, the detection methods mainly focus on identification of toxin A, toxin B and binary toxin CDT in samples, preferably stool samples.

Testing methods include tissue culture cytotoxicity assays and/or *Clostridioides difficile* culture identification and/or enzyme immunoassays (EIAs) for toxins A and/or B, and EIAs for glutamate dehydrogenase (GDH, antigen of the C. difficile cell wall). Whereas the tissue culture cytotoxicity assay and culture identification are labor and time consuming, as the results are only obtained within 24 to 96 hours, EIA tests results show comparatively low specificity.

Direct stool culture and cell cytotoxin neutralization assay (CCNA) tests are currently the reference standard methods for the diagnosis of CDI. However, both methods are laborious, time consuming, and must be performed by qualified, trained personnel.

Recently, polymerase chain reaction tests have been made available. These methods detect relevant genes with high sensitivity, specificity, and low turnaround time. One test relates to the use of a set of primers that can detect a set of three genes, namely tcdA and tcdB, which respectively code toxins A and B, as well as tcdC, which is present in mutated form in hyper-virulent *Clostridioides difficile* strains, and so allows overexpression of toxin A and B (Patent Document 1). This method enables the detection of hyper-virulent *Clostridioides difficile* strains, in particular *Clostridioides difficile* strains belonging to the B1/NAP1/027 group. However, the test requires many process steps and the use of different laboratory equipment, which makes its set up complex, expensive and time consuming.

Commercially available products for *Clostridium difficile* diagnosis include the BD GeneOhm Cdiff (BD Diagnostics; San Diego, CA, USA), Prodesse ProGastro Cd (Gen-Probe Inc.; San Diego, CA, USA), Xpert *Clostridium difficile* (Cepheid; Sunnyvale, CA, USA), and illumigene *Clostridium difficile* (Meridian Biosciences; Cincinnati, OH, USA) real-time PCR tests. All the tests target the tcdB gene, except for the illumigene *Clostridium difficile* test, which targets the tcdA gene. The Xpert *Clostridium difficile* / Epi (Cepheid) is additionally capable of detecting the binary toxin. Compared to the CCNA and/or toxigenic culture, the PCR assays were reported with enhanced sensitivities and specificities ranging from 77% to 100% and 93% to 100%, respectively (Non-Patent Literature 3).

Nevertheless, there still exists a need to provide an alternative method to determine the presence of a hyper-virulent *Clostridioides difficile* strain, in particular a hyper-virulent *Clostridioides difficile* strains belonging to the B1/NAP1/027 group, wherein the method preferably exhibits a suitable sensitivity and/or specifity, is simple, time and/or cost saving.

Thus, the problem addressed by the present invention is to provide a method for determining the presence of a hyper-virulent *Clostridioides difficile* strains belonging to the B1/NAP1/027 group, preferably with a suitable specificity and / or sensivity and more preferably being simple, time and/or cost saving.

### CITATION LIST

Non-Patent Literature 1: *"*Reclassification of Clostridium difficile as Clostridioides difficile (Hall and O'Toole 1935) Prévot 1938", Lawson et al., Anaerobe 2016; 40: 95-99
Non-Patent Literature 2: *"*Comparison of Seven Techniques for Typing International Epidemic Strains of Clostridium difficile: Restriction Endonuclease Analysis, Pulsed-Field Gel Electrophoresis, PCR-Ribotyping, Multilocus Sequence Typing, Multilocus Variable-Number Tandem-Repeat Analysis, Amplified Fragment Length Polymorphism, and Surface Layer Protein A Gene Sequence Typing", Killgore G, Thompson, et al., J Clin Microbiol 46 (2) February 2008, p. 431-437
Non-Patent Literature 3: *"*Multiplex Real-Time PCR Method for Simultaneous Identification and Toxigenic Type Characterization of Clostridium difficile From Stool Samples", Abdullah Kilic et al., Annals of Lab Medicine, 2015 May; 35(3), p. 306-313

Patent Literature 1: WO 2010/116290 A1

### SUMMARY OF THE INVENTION:

One or more problems addressed by the present invention is / are solved by the subject matter of the independent claims, namely the primer pair or oligonucleotide probe for hybridizing to at least a portion of a chloramphenicol acetyltransferase gene coded in a transposon of family CTn-027 of a hyper-virulent *Clostridioides difficile* strain belonging to the B1/NAP1/027 group, the oligonucleotide set comprising the primer pair or oligonucleotide probe, the kit for detecting the presence of a hyper-virulent strain of Clostridioides difficile belonging to the B1/NAP1/027 group in a sample comprising the primer pair or oligonucleotide probe, and the method for determining the presence of a hyper-virulent *Clostridioides difficile* strain belonging to the B1/NAP1/027 group. Advantages (preferred embodiments) are set out in the detailed description hereinafter and/or the accompanying figures as well as in the dependent claims.

Accordingly, a first aspect of the present invention relates to a primer pair for hybridizing to at least a portion of a chloramphenicol acetyltransferase gene coded in a transposon of family CTn-027 of a hyper-virulent *Clostridioides difficile* strain of the B1/NAP1/027 group, characterized in that the primer comprises or consists of
a. A first isolated oligonucleotide a) comprising or consisting of at least 88 %, 89 %, 90 %, 91 %, 92 %, 93 %, 94 %, 95 %, 96 %, 97 %, 98 %, 99 % or 100 % sequence identity to SEQ ID No. 1, wherein the oligonucleotide comprises at least 10 nucleotides, or an isolated oligonucleotide comprising or consisting of a complimentary oligonucleotide sequence to the isolated oligonucleotide comprising or consisting of at least 88 %, 89 %, 90 %, 91 %, 92 %, 93 %, 94 %, 95 %, 96 %, 97 %, 98 %, 99 % or 100 % sequence identity to SEQ ID No. 1, and
b. A second isolated oligonucleotide b) hybridizing to at least a portion of a chloramphenicol acetyltransferase gene coded in a transposon of family CTn-027 of a hyper-virulent *Clostridioides difficile* strain of the group B1/NAP1/027, preferably comprising or consisting of at least 90 %, 91 %, 92 %, 93 %, 94 %, 95 %, 96 %, 97 %, 98 %, 99 % or 100 % sequence identity to SEQ ID No. 2, wherein the oligonucleotide comprises at least 10 nucleotides, or an isolated oligonucleotide comprising or consisting of a complimentary oligonucleotide sequence to the oligonucleotide comprising or consisting of at least 90 %, 91 %, 92 %, 93 %, 94 %, 95 %, 96 %, 97 %, 98 %, 99 % or 100 % sequence identity to SEQ ID No. 2.

The primer pair according to the first inventive aspect is generally used for hybridizing to and amplifying of at least a portion of a chloramphenicol acetyltransferase gene coded in a transposon of family CTn-027 of a hyper-virulent *Clostridioides difficile* strain of the B1/NAP1/027 group, preferably of a chloramphenicol acetyltransferase gene according to SEQ ID No. 3, more preferably of a chloramphenicol acetyltransferase gene according to SEQ ID No. 4.

A second aspect of the present invention relates to an oligonucleotide probe for hybridizing to at least a portion of a chloramphenicol acetyltransferase gene coded in a transposon of family CTn-027 of a hyper-virulent *Clostridioides difficile* strain of the B1/NAP1/027 group, characterized in that the oligonucleotide probe comprises or consists of an isolated oligonucleotide a) comprising or consisting of at least 88 %, 89 %, 90 %, 91 % , 92 % , 93 %, 94 %, 95 %, 96 %, 97 %, 98 %, 99 % or 100 % sequence identity to SEQ ID No. 1, wherein the oligonucleotide comprises at least 10 nucleotides, or an isolated oligonucleotide comprising or consisting of a complimentary oligonucleotide sequence to the isolated oligonucleotide comprising or consisting of at least 88 %, 89 %, 90 %, 91 %, 92 %, 93 %, 94 %, 95 %, 96 %, 97 %, 98 %, 99 % or 100 % sequence identity to SEQ ID No. 1.

The oligonucleotide probe according to the second inventive aspect is generally used for hybridizing to and detecting of at least a portion of a chloramphenicol acetyltransferase gene coded in a transposon of family CTn-027 of a hyper-virulent *Clostridioides difficile* strain of the B1/NAP1/027 group, preferably of a chloramphenicol acetyltransferase gene according to SEQ ID No. 3, more preferably of a chloramphenicol acetyltransferase gene according to SEQ ID No. 4. The oligonucleotide probe may be any suitable probe, such as a hybridization probe, hydrolysis probe, etc.

A third aspect of the present invention relates to an oligonucleotide set for use in a nucleic acid amplification process or a nucleic acid detection process for determining the presence of a hyper-virulent *Clostridioides difficile* strain of the B1/NAP1/027 group in a sample, characterized in that the oligonucleotide set comprises the inventive primer pair of the first inventive aspect or the inventive oligonucleotide probe of the second inventive aspect, and one or more additives.

A fourth aspect of the present invention relates to a kit for detecting the presence of a hyper-virulent strain of *Clostridioides difficile* of the B1/NAP1/027 group in a sample comprising the inventive primer pair of the first inventive aspect, the inventive oligonucleotide probe of the second inventive aspect and/or the inventive oligonucleotide set of the third inventive aspect, characterized in that the oligonucleotide a) and oligonucleotide b), if present, hybridize under stringent conditions to at least part of SEQ ID No. 3.

A fifth aspect of the present invention relates to a method of determining the presence of a hyper-virulent strain of *Clostridioides difficile* of the B1/NAP1/027 group in a sample, the method comprises or consists of:
a. performing a nucleic acid hybridization and amplification reaction comprising a denatured nucleotide extract of the sample as a template and an inventive primer pair according to the first inventive aspect or an inventive oligonucleotide set according to the third inventive aspect or an inventive kit according to the fourth inventive aspect in order to generate an amplicon of at least a portion of a chloramphenicol acetyltransferase gene coded in a transposon of family CTn-027 of a hyper-virulent *Clostridioides difficile* strain of the B1/NAP1/027 group,
b. detecting the amplicon generated in step a), and
c. determining the presence of a hyper-virulent strain of *Clostridioides difficile* of the B1/NAP1/027 group in the sample as a function of the detected amplicon in step b).

The inventive aspects of the present invention as disclosed hereinbefore can comprise any possible (sub-)combination of the preferred inventive embodiments as set out in the dependent claims or as disclosed in the following detailed description and/or in the accompanying figures, provided the resulting combination of features is reasonable to a person skilled in the art.

### BRIEF DESCRIPTION OF THE DRAWINGS:

Further characteristics and advantages of the present invention will ensue from the accompanying drawings, wherein
**Fig. 1** represents graphs on growth curves in comparative PCR study for detection *Clostridioides difficile* strains of group 1 (R2091 strain of B1/NAP1/027 group), group 2 (ATCC 9689 strain, closely related to B1/NAP1/027 group) and group 3 (630 strain, no NAP1 group).
**Fig. 2** represents graphs on melting curves in comparative PCR study for detection *Clostridioides difficile* strains of group 1 (R2091 strain of B1/NAP1/027 group), group 2 (ATCC 9689 strain, closely related to B1/NAP1/027 group) and group 3 (630 strain, no NAP1 group).

### DETAILED DESCRIPTION OF THE INVENTION:

The inventors of the present invention have found out that the inventive primer pair of the first inventive aspect and the inventive oligonucleotide probe of the second inventive aspect specifically enable to determine the presence of a hyper-virulent *Clostridioides difficile* strain of the B1/NAP1/027 group, whereas strains closely related to B1/NAP1/027 as well as other strains are truly not determined. The specificity is due to the fact that the inventive primer pair and the inventive oligonucleotide probe have been optimized to specifically amplify and detect respectively to such a part of the chloramphenicol acetyltransferase gene coded in a transposon of family CTn-027 as target, which is specific for hyper-virulent *Clostridioides difficile* strains of the B1/NAP1/027 group and is not present in other closely related strains. Thus, the inventive primer pair and the inventive oligonucleotide probe show a high specifity for *Clostridioides difficile* strains belonging to the B1/NAP1/027 group. Furthermore, the inventive detection method of the fourth inventive aspect provides a simple and highly sensible detection method for hyper-virulent *Clostridioides difficile* strains, more preferably hyper-virulent *Clostridioides difficile* strains belonging to the B1/NAP1/027 group (for detailed information see Example section).

According to a preferred embodiment of all aspects of the present invention, the oligonucleotide coding the chloramphenicol acetyltransferase gene in a transposon of family CTn-027 of a hyper-virulent *Clostridioides difficile* strain comprises or consists of SEQ ID No. 3. The oligonucleotides represented in SEQ ID No. 3 refer to the set of oligonucleotides representing the full chloramphenicol acetyltransferase gene in the transposon of family CTn-027 of a hyper-virulent *Clostridioides difficile* strain. According to a further preferred embodiment of the first inventive aspect, the oligonucleotides coding the chloramphenicol acetyltransferase gene in a transposon of family CTn-027 of a hyper-virulent *Clostridioides difficile* strain comprise or consist of SEQ ID No. 4, as this sequence is specific for *Clostridioides difficile* strains belonging to the B1/NAP1/027 group.

In the context of the present invention, the expression *"an additionally or alternatively further preferred embodiment"* or *"an additionally or alternatively preferred embodiment"* or *"an additional or alternative way of configuring this preferred embodiment"* means that the feature or feature combination disclosed in this preferred embodiment can be combined in addition to or alternatively to the features of the inventive subject matter including any preferred embodiment of each of the inventive aspects, provided the resulting feature combination is reasonable to a person skilled in the art.

As already mentioned in the summary section of the invention, the inventive primer pair according to the first inventive aspect is generally used for hybridizing and amplifying of at least a portion of a chloramphenicol acetyltransferase gene coded in a transposon of family CTn-027 of a hyper-virulent *Clostridioides difficile* strain of the B1/NAP1/027 group, whereas the inventive oligonucleotide probe according to the second inventive aspect is generally used for hybridizing and detecting of at least a portion of a chloramphenicol acetyltransferase gene coded in a transposon of family CTn-027 of a hyper-virulent *Clostridioides difficile* strain of the B1/NAP1/027 group.

The isolated oligonucleotide a) of the inventive primer pair of the first inventive aspect or the inventive oligonucleotide probe of the second inventive aspect comprises or consists of at least 88 %, 89 %, 90 %, 91 %, 92 %, 93 %, 94 %, 95 %, 96 %, 97 %, 98 %, 99 % or 100 % sequence identity to SEQ ID No. 1, wherein the oligonucleotide comprises at least 10 nucleotides. Alternatively, an isolated oligonucleotide can be used as oligonucleotide a), which comprises or consists of a complimentary oligonucleotide sequence to the oligonucleotide comprising or consisting of at least 88 %, 89 %, 90 %, 91 %, 92 %, 93 %, 94 %, 95 %, 96 %, 97 %, 98 %, 99 % or 100 % sequence identity to SEQ ID No. 1. In order to increase the hybridization accuracy, the isolated oligonucleotide a) may comprise 15 or more, more preferably 20 or more nucleotides.

The second isolated oligonucleotide b) of the inventive primer pair is generally selected from primer oligonucleotides hybridizing to at least a portion of the chloramphenicol acetyltransferase gene coded in a transposon of family CTn-027 of a hyper-virulent *Clostridioides difficile* strain of the B1/NAP1/027 group, preferably of SEQ ID No. 3, more preferably SEQ ID No. 4. More preferably, the second isolated oligonucleotide b) of the inventive primer pair comprises or consists of at least 90 %, 91 %, 92 %, 93 %, 94 %, 95 %, 96 %, 97 %, 98 %, 99 % or 100 % sequence identity to SEQ ID No. 2, wherein the oligonucleotide comprises at least 10 nucleotides. Alternatively, an isolated oligonucleotide can be used as oligonucleotide b), which comprises or consists of a complimentary oligonucleotide sequence to the oligonucleotide comprising or consisting of at least 90 %, 91 %, 92 %, 93 %, 94 %, 95 %, 96 %, 97 %, 98 %, 99 % or 100 % sequence identity to SEQ ID No. 2. In order to increase the hybridization accuracy, the isolated oligonucleotide b) may preferably comprise 15 or more, more preferably 20 or more nucleotides.

According to an exemplified embodiment, the inventive primer pair comprises or consists of an isolated oligonucleotide a) according to SEQ ID No. 1 or its complimentary sequence and an isolated oligonucleotide b) according to SEQ ID 2 or its complimentary sequence.

According to an additionally or alternatively further preferred embodiment of the first inventive aspect, the oligonucleotide a) and/or oligonucleotide b) of the inventive primer pair may independently from each other comprise one or more added functionalities, e.g. quencher and/or fluorophores in particular detectable by fluorescence resonance energy transfer (FRET), radioisotopes and/or may comprise one or more modified nucleotides, e.g. locked nucleic acid (LNA). Such added functionalities may enhance the hybridization of the inventive primer pair to the target and/or detection of the inventive oligonucleotide probe.

According to an exemplified embodiment, the inventive oligonucleotide probe comprises of consists of an isolated oligonucleotide a) according to SEQ ID No. 1 or its complimentary sequence and one or more suitable detection functionalities for detection of *Clostridioides difficile* of the B1/NAP1/027 group, in particular for quantitative detection of *Clostridioides difficile* of the B1/NAP1/027 group, such as quencher and/or fluorophores moieties, more preferably a fluorophore moiety and a quencher moiety at the 5' end and at the 3' end of the oligonucleotide probe respectively.

The genome of a reference strain R20291 of *Clostridioides difficile* of the B1/NAP1/027 group has been chosen as an example in order to optimize the inventive primer pair of the first inventive aspect and the inventive oligonucleotide probe of the second inventive aspect. The fully sequenced genome is deposited with gene accession no: FN545816.1.

As set out in detail in the example section (executive examples 1 and 2), the inventive primer pair facilitates to specifically hybridize to and to amplify hyper-virulent *Clostridioides difficile* strains of the B1/NAP1/027 group (group 1), whereas the inventive primer pair does not hybridize to and amplify strains closely related to B1/NAP1/027 as determined by ribotyping (group 2) as well as other *Clostridioides difficile* strains, such as 630 (group 3). Thus, the inventive primer pair is highly specific for the determination of hyper-virulent *Clostridioides difficile* strains of the B1/NAP1/027 group in comparison to closely related strains of group 2 or other strains of group 3.

The same is true for the inventive oligonucleotide probe, which facilitates to specifically hybridize to and to detect hyper-virulent *Clostridioides difficile* strains of the B1/NAP1/027 group (group 1), whereas the inventive oligonucleotide probe does not hybridize to and detect strains closely related to B1/NAP1/027 as determined by ribotyping (group 2) as well as other *Clostridioides difficile* strains, such as 630 (group 3). Thus, the inventive oligonucleotide probe is highly specific for the detection of hyper-virulent *Clostridioides difficile* strains of the B1/NAP1/027 group in comparison to closely related strains of group 2 or other strains of group 3.

All features and embodiments disclosed with respect to the first and second aspects of the present invention are combinable alone or in (sub-)combination with each other and with the third to fifth aspects of the present invention respectively including each of the preferred embodiments thereof, provided the resulting combination of features is reasonable to a skilled person.

According to the third aspect of the present invention, an oligonucleotide set is provided for use in a nucleic acid amplification process for determining the presence of a hyper-virulent *Clostridioides difficile* strain of the B1/NAP1/027 group in a sample, characterized in that the oligonucleotide set comprises the inventive primer pair of the first inventive aspect or the inventive oligonucleotide probe of the second inventive aspect, and one or more additives. Suitable additives, such as reagents, in particular solvents etc. are readily available in the prior art.

All features and embodiments disclosed with respect to the third aspect of the present invention are combinable alone or in (sub-)combination with each of the first, second, fourth and fifth aspects of the present invention respectively including each of the preferred embodiments thereof, provided the resulting combination of features is reasonable to a skilled person.

According to the fourth aspect of the present invention a kit for detecting the presence of a hyper-virulent strain of *Clostridioides difficile* of the B1/NAP1/027 group in a sample comprising the inventive primer pair according to the first inventive aspect, the inventive oligonucleotide probe according to the second inventive aspect and/or the oligonucleotide set according to the third inventive aspect, characterized in that the inventive oligonucleotide a) and oligonucleotide b), if present, hybridize under stringent conditions to at least part of SEQ ID No. 3, preferably at least part of SEQ ID No. 4.

According to an additionally or alternatively preferred embodiment the inventive kit for detecting the presence of a hyper-virulent strain of *Clostridioides difficile* of the B1/NAP1/027 group in a sample further comprises one, two, three, four or more constituents (reactants) selected from the group of enzymes, such as polymerase (e.g. taq DNA polymerase), ligase, helicase, or recombinase; deoxynucleotides; control template; buffer and additives. Preferably, the buffer is dissolved in water, preferably nuclease free water, and comprises or consists of one or more constituents selected from the group of salts, such as potassium chloride (KCI), ammonium sulfate (NH₄)₂SO₄, tris(hydroxymethyl)aminomethane hydrochloride (Tris-HCI), or proteins, such as albumin including Bovine Serum Albumin; and/or the additives comprise or consist of one, two or more constituents selected from the group of co-factors, such as magnesium chloride (MgCl₂); dimethyl sulfoxide (DMSO); fluorophores, such as fluorescent dyes intercalating into double stranded nucleic acid strands (e.g. SYBR Green I); and quenchers.

One or more of the constituents in the inventive kit may be present in a concentrated form, i.e. need to be diluted, preferably with nuclease free water, prior to their application in the amplification reaction. As an example one or more of the constituents may be present in a two-fold concentrated form, i.e. the respectively concentrated constituents need to be diluted to twice of the concentrated volume.

As an example, the stringent condition in the inventive kit for detecting the presence of a hyper-virulent strain of *Clostridioides difficile* of the B1/NAP1/027 group comprises nuclease free water and 0.05 to 0.1 U/µl polymerase, preferably taq DNA polymerase, 10 to 200 mM Tris-HCI, 0.5 to 5 mM, preferably 3 mM MgCl₂, 50 to 500, preferably 50 mM KCI and a pH in the range of 6.8 to 9.1, preferably pH of 8.4 to 8.8. In addition, a suitable amount of detection agent, such as fluorophore, preferably a fluorescent dye intercalating into double stranded DNA is also present in the kit.

All features and embodiments disclosed with respect to the fourth aspect of the present invention are combinable alone or in (sub-)combination with each of the first, second, third and fifth aspects of the present invention respectively including each of the preferred embodiments thereof, provided the resulting combination of features is reasonable to a skilled person.

According to the fifth aspect of the present invention, a method of determining the presence of a hyper-virulent strain of *Clostridioides difficile* of the B1/NAP1/027 group in a sample is provided. As already mentioned above, the inventive detection method provides a simple, and thus, cost effective detection method that exhibits at the same time high sensitivity.

The inventive method comprises step a) performing a nucleic acid hybridization and amplification reaction comprising a denatured nucleotide extract of the sample as a template and an inventive primer pair or the inventive oligonucleotide set or the inventive kit in order to generate an amplicon of at least a portion of a chloramphenicol acetyltransferase gene coded in a transposon of family CTn-027 of a hyper-virulent *Clostridioides difficile* strain of the B1/NAP1/027 group. The denatured nucleotide extract of the sample may be commercially available or may be produced from the specimen sample according to suitable methods of the prior art, such as described as *"*Optimized Protocol for Simple Extraction of High-Quality Genomic DNA from Clostridium difficile for Whole-Genome Sequencing" (James Heng Chiak Sim et al., Journal of Clinical Microbiology, July 2015, Vol. 53, Number 7, page. 2329 - 2331). Any suitable Kit may be used for DNA isolation, such as Genomic Mini Kit (A&A Biotechnology). Diagnosis and typing of human *Clostridioides difficile* infection may be conducted by suitable standard operating procedures (SOPs), such as *"*Laboratory procedures for diagnosis and typing of human Clostridium difficile infection" issued by the European Center for Disease Prevention and Control 19 October 2018.

According to an additional or alternative preferred embodiment, the amplification reaction is selected from the group consisting of a polymerase chain reaction (PCR), such as Real-Time PCR (qPCR), a ligase chain reaction (LCR), ligase detection reaction (LDR), multiplex PCR-ligase detection reaction assay, multiplex ligation dependent probe amplification (MLPA), multiplex oligonucleotide ligation PCR (MOL-PCR), Isothermal DNA amplification, Rolling Circle Amplification (RCA), Helicase-Dependent Amplification (HAD), Signal-Mediated Amplification of RNA Technology (SMART), Recombinase Polymerase Amplification (RPA), Polymerase Spiral Reaction (PSR), Strand displacement amplification. In particular, real-Time PCR (qPCR) not only allows qualitative, but also quantitative detection of the hyper-virulent *Clostridioides difficile* strain of the B1/NAP1/027 group in the sample.

The ligase chain reaction (LCR) is in particular suitable in case in addition to the inventive primer pair, a second primer pair for a second template strand is used.

The ligase detection reaction (LDR) is a modified LCR technique, where only two oligonucleotides, instead of four oligonucleotides bind adjacently on one target strand.

With respect to multiplex ligation dependent probe amplification (MLPA) two oligonucleotide probes bind adjacently on a target sequence and are ligated only in presence of their complementary target DNA.

Multiplex oligonucleotide ligation PCR (MOL-PCR) is similar to MLPA, but a flow cytometric device is used to identify each bead to measure fluorescence intensity for each bead-probe complex. For MOL-PCR the reverse primer and not the forward primer is fluorescently labelled.

Rolling Circle Amplification (RCA) is an anisothermal enzymatic process that uses DNA or RNA polymerases to produce single stranded DNA (ssDNA) or RNA molecules, which are a connection in series of complementary units of a template.

Helicase-Dependent Amplification (HAD) follows replication fork mechanism for amplification, which utilizes DNA helicase for unwinding activity Helicase separates the duplex DNA into single-stranded DNA for in vitro amplification of target DNA. The amplification is extended by exonuclease deficient DNA polymerases at a single constant temperature. The HAD products are detected by gel electrophoresis.

Signal-Mediated Amplification of RNA Technology (SMART) is based on signal amplification and it does not depend on copying of target sequences. The SMART method consists of two single-stranded oligonucleotide probes that are annealed to a specific target sequence and then it forms a three-way junction (3WJ) structure. The reaction is carried out by three enzymes like Bst DNA Polymerase, T7 RNA polymerase and DNA polymerase at a constant temperature of 41°C. The amplicons are detected by ELOSA and real-time.

Recombinase Polymerase Amplification (RPA) is an isothermal method and amplifies target DNA sequences by using a DNA polymerase, recombinase and DNA-binding proteins at a temperature of 37 - 42°C. The amplified products are detected by the fluorophore, quencher groups and microfluidic devices.

Polymerase Spiral Reaction (PSR) can be finished within 1 h with a high sensitivity (up to 109 copies) and high specificity to detect the target. The PSR method exploits the advantages of PCR in which only one pair of primers is needed and isothermal amplification techniques like LAMP assay.

Strand displacement amplification is based upon the ability of HincII to nick the unmodified strand of a hemiphosphorothioate form of its recognition site, and the ability of exonuclease deficient klenow (exo- klenow) to extend the 3'-end at the nick and displace the downstream DNA strand. Exponential amplification results from coupling sense and antisense reactions in which strands displaced from a sense reaction serve as target for an antisense reaction and vice versa.

The inventive determination method further comprises as step b) detecting the amplicon generated in step a). According to an additional or alternative preferred embodiment, the amplicon is detected by use of a non-specific detection method, such as detection of fluorescence with suitable means. As an example, a fluorescence dye non-specifically intercalating into the double stranded nucleic acid strand of the amplicon may be detected. SYBR Green I, is an example of a commonly used fluorescent DNA binding dye, which binds all double-stranded DNA and detection is monitored by measuring the increase in fluorescence throughout the cycle. SYBR Green I has an excitation and emission maxima of 494 nm and 521 nm, respectively. Specificity of Sigma's SYBR based QPCR detection is greatly enhanced by the incorporation of a hot-start mediated taq polymerase, JumpStart Taq. Alternatively, fluorophores and quenchers as FRET pairs, e.g. in TaqMan assays or in techniques using hybridization probes can be used. As an example, the inventive oligonucleotide probe of the second inventive aspect can be used to detect the generated amplicon of step a).

The method further comprises as step c) determining the presence of a hyper-virulent strain of *Clostridioides difficile* of the B1/NAP1/027 group in the sample, in case the amplicon is positively detected in step b) determining the presence of a hyper-virulent strain of *Clostridioides difficile* of the B1/NAP1/027 group in the sample as a function of the detected amplicon in step b). In other words, in case the amplicon of at least a portion of a chloramphenicol acetyltransferase gene coded in the transposon of family CTn-027 of a hyper-virulent *Clostridioides difficile* strain of the B1/NAP1/027 group is generated in step a) and detected in step b), then the presence of a hyper-virulent strain of *Clostridioides difficile* of the B1/NAP1/027 group is determined in step c). This means in turn, that in case the amplicon of at least a portion of a chloramphenicol acetyltransferase gene coded in the transposon of family CTn-027 of a hyper-virulent *Clostridioides difficile* strain of the B1/NAP1/027 group is not generated in step a) and, thus, not detected in step b), then the presence of a hyper-virulent strain of *Clostridioides difficile* of the B1/NAP1/027 group is not determined in step c). As set out in the example section, the inventive method for determining the presence of a hyper-virulent strain of *Clostridioides difficile* of the B1/NAP1/027 group is highly specific. This means, that the inventive primer pair, when subjected to *Clostridioides difficile* strains closely related to the B1/NAP1/027 group or other strains, do not generate the amplicon in step a) and, thus, those strains are truly not determined as *Clostridioides difficile* of the B1/NAP1/027 group. Moreover, in view of the use of the amplification reaction, the inventive method is also highly sensitive as well as simple, time and cost effective.

In general, the sample to be detected by the inventive method of the fifth inventive aspect may be any suitable specimen. According to an additional or alternative preferred embodiment, the sample for determining the presence of a hyper-virulent strain of *Clostridioides difficile* of the B1/NAP1/027 group is selected from the group consisting of a human sample, an animal sample, an object sample or a food sample, more preferably selected from stool sample, a smear test sample, a body fluid sample, or a tissue sample, respectively from human or animal.

All features and embodiments disclosed with respect to the fifth aspect of the present invention are combinable alone or in (sub-)combination with each of the first, second, third and fourth aspects of the present invention respectively including each of the preferred embodiments thereof, provided the resulting combination of features is reasonable to a skilled person.

### EXAMPLES:

Further characteristics and advantages of the present invention will ensue from the following description of example embodiments of the inventive aspects with reference to the accompanying drawings.

All of the features disclosed hereinafter with respect to the example embodiments can alone or in any sub-combination be respectively combined with features of the different aspects of the present invention including features of preferred embodiments thereof, provided the resulting feature combination is reasonable to a person skilled in the art.

### EXECUTIVE EXAMPLE 1: IN SILICO COMPARISON OF INVENTIVE PRIMER PAIR / INVENTIVE PROBE SELECTIVITY FOR B1/NAP1/027 STRAINS VERSUS OTHER STRAINS OF CLOSTRIDIOIDES DIFFICILE

To show specificity of the inventive primer pair and inventive oligonucleotide probe, the following alignment was conducted. First, the target sequence of the chloramphenicol acetyltransferase gene according to SEQ ID No. 3 as present in *Clostridioides difficile* strain R2091 complete genome was entered into Basic Local Alignment Search Tool (BLAST) in order to align target sequence of *Clostridioides difficile* of the B1/NAP1/027 group to any known homologues. The results obtained from the BLAST alignment were grouped as follows:
Group 1 as set out in table 1 below contains strains that have 100% query cover and identity with target sequence, all strains are recognized as specimens that belong to *Clostridioides difficile* of the B1/NAP1/027 group and are deemed to exhibit true positive scores in detection step b) of the inventive method.

**Table 1:**

| ***Clostridioides difficile strain*** | **Max score** | **Total score** | **Query cover** | **E value** | **% Identity** | **Accession** |
|---|---|---|---|---|---|---|
| *Clostridioides difficile* strain 020711 | 1192 | 1192 | 100% | 0.0 | 100.00 | CP028530.1 |
| *Clostridioides difficile* strain 020696 | 1192 | 1192 | 100% | 0.0 | 100.00 | CP028528.1 |
| *Clostridioides difficile* strain 020695 | 1192 | 1192 | 100% | 0.0 | 100.00 | CP028527.1 |
| *Clostridioides difficile* strain 020688 | 1192 | 1192 | 100% | 0.0 | 100.00 | CP028526.1 |
| *Clostridioides difficile* strain 020474 | 1192 | 1192 | 100% | 0.0 | 100.00 | CP028523.1 |
| *Clostridioides difficile* strain 020482 | 1192 | 1192 | 100% | 0.0 | 100.00 | CP028525.1 |
| *Clostridioides difficile* strain DSM 28196, complete genome | 1192 | 1192 | 100% | 0.0 | 100.00 | CP012320.1 |
| *Clostridioides difficile* strain DSM 27640, complete genome | 1192 | 1192 | 100% | 0.0 | 100.00 | CP011848.1 |
| *Clostridioides difficile* strain DSM 27638, complete genome | 1192 | 1192 | 100% | 0.0 | 100.00 | CP011846.1 |
| *Clostridioides difficile* strain 10-00253, complete genome | 1192 | 1192 | 100% | 0.0 | 100.00 | CP026598.1 |
| *Clostridioides difficile* strain 10-00078, complete genome | 1192 | 1192 | 100% | 0.0 | 100.00 | CP026597.1 |
| *Clostridioides difficile* strain 09-00072, complete genome | 1192 | 1192 | 100% | 0.0 | 100.00 | CP026599.1 |
| *Clostridioides difficile* strain 10-00071, complete genome | 1192 | 1192 | 100% | 0.0 | 100.00 | CP026596.1 |
| *Clostridioides difficile* strain 12-00011, complete genome | 1192 | 1192 | 100% | 0.0 | 100.00 | CP026595.1 |
| *Clostridioides difficile* strain 08-00495, complete genome | 1192 | 1192 | 100% | 0.0 | 100.00 | CP026594.1 |
| *Clostridioides difficile* strain CD-17-01474, complete genome | 1192 | 1192 | 100% | 0.0 | 100.00 | CP026591.1 |
| *Clostridioides difficile* strain CD-10-00484, complete genome | 1192 | 1192 | 100% | 0.0 | 100.00 | CP026592.1 |
| *Clostridioides difficile* strain 1200008, complete genome | 1192 | 1192 | 100% | 0.0 | 100.00 | CP026593.1 |
| *Clostridioides difficile* strain R0104a | 1192 | 1192 | 100% | 0.0 | 100.00 | CP025044.1 |
| *Clostridium difficile* BI1, complete genome | 1192 | 1192 | 100% | 0.0 | 100.00 | FN668941.1 |
| *Clostridium difficile* complete genome, strain 2007855 | 1192 | 1192 | 100% | 0.0 | 100.00 | FN668941.1 |
| *Clostridium difficile* R2091 complete genome | 1192 | 1192 | 100% | 0.0 | 100.00 | FN545816.1 |
| *Clostridium difficile* CD196 complete genome, strain CD196 | 1192 | 1192 | 100% | 0.0 | 100.00 | FN538970.1 |

Group 2 as set out in table 2 below contains strains with lower coefficient in query cover and identity, hence has some difference in sequence, such as deletions or inversions, all strains are recognized as specimens that do not belong to *Clostridioides difficile* of the B1/NAP1/027 group, but are deemed to be closely related. This group has been chosen, as it was expected that they might produce false positive scores in the detection step b) of the inventive method.

**Table 2:**

| ***Clostridioides difficile strain*** | **Max score** | **Total score** | **Query cover** | **E value** | **% Identity** | **Accession** |
|---|---|---|---|---|---|---|
| *Clostridioides difficile* strain DSM 29745, complete genome | 922 | 922 | 99% | 0.0 | 92.56 | CP019857.1 |
| *Clostridioides difficile* strain FDAARGOS_267, complete genome | 922 | 922 | 99% | 0.0 | 92.56 | CP028528.1 |
| *Clostridioides difficile* strain AK | 922 | 922 | 99% | 0.0 | 92.56 | CP028527.1 |
| *Clostridioides difficile* strain Z31 | 922 | 922 | 99% | 0.0 | 92.56 | CP028526.1 |
| *Clostridioides difficile* ATCC 9689 = DSM 1296 | 922 | 922 | 99% | 0.0 | 92.56 | CP028523.1 |
| *Clostridium difficile* BI9 strain | 922 | 922 | 99% | 0.0 | 92.56 | CP028525.1 |
| *Clostridioides difficile* strain DSM 27639 | 911 | 911 | 99% | 0.0 | 92.24 | CP012320.1 |

Group 3 comprises all other strains, such as Clostridium difficile 630 strain, that do not show homology to the target sequence.

### EXECUTIVE EXAMPLE 2: PCR STUDY FOR COMPARISON OF INVENTIVE PRIMER PAIR SELECTIVITY FOR B1/NAP1/027 STRAINS VERSUS OTHER STRAINS OF CLOSTRIDIOIDES DIFFICILE

### Protocol of sample preparation:

1. *C. difficile* ATCC 9689 bacteria were grown on Columbia Agar with 5% Sheep Blood plates (Oxoid; catalog no. PB5039A) at 37°C under anaerobic conditions (AnaeroGen Compact, Oxoid; catalog no. AN0010C and AN0020D) for 48 hours.
2. *C. difficile* colonies were scraped from plates with inoculating loop and suspended in 10 mM Tris-HCI pH 8.0 buffer to equal 1.0 McFarland.
3. For DNA extraction a 100 µl volume of a 1.0 McFarland suspension per sample were used.
4. Bacterial DNA was extracted using a Genomic Mini Kit (A&A Biotechnology) according to the manufacturer protocol.

In analogy, the sample preparation is conducted with respect to *C. difficile* 630 strain. With respect to *C. difficile* R20291 strain a DNA source was provided as sample material.

Respective Bacteria culture and/or DNA material can be obtained from Public Health England, Leibniz Institute DSMZ-German Collection of Microorganisms and Cell Cultures GmbH, and the ATCC.

### Reaction mixture for real time-PCR on LightCycler 480 (ROCHE):

10 µl RT HS-PCR Mix SYBR® A (A&A Biotechnology)
8 µl H₂O sterile water, nucleases-free. Treated with DEPC. (A&A Biotechnology)
1 µl Primer mix (solution in 10mM Tris-HCI pH 8.0) (2.5 µM each)
1 µl DNA (in negative control - 1 µl of sterile water)

### PCR reaction conditions:

Initial denaturation: 95°C 5 min
45 cycles:
   Step 1 - 95°C 15 s
   Step 2 - 55°C 25 s
   Step 3 - 72°C 15 s
Melting - 65 - 97°C

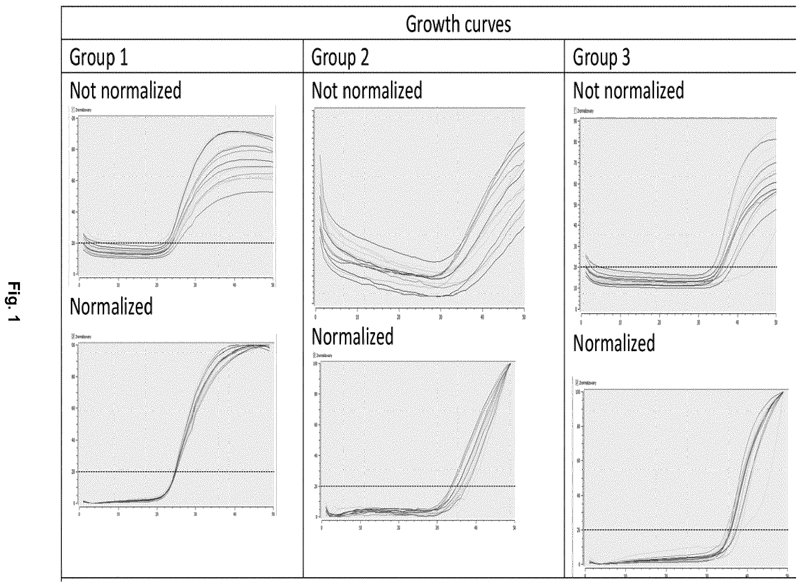

Fig. 1 shows graphs on growth curves in the comparative PCR assay for detection of *Clostridioides difficile* strains of group 1 (R2091 strain of B1/NAP1/027 group), group 2 (ATCC 9689 strain, closely related to B1/NAP1/027 group) and group 3 (630 strain, no NAP1 group), respectively. When comparing the growth curves of the respective groups it is apparent, that for group 1 the exponential growth starts earlier than for groups 2 and 3.

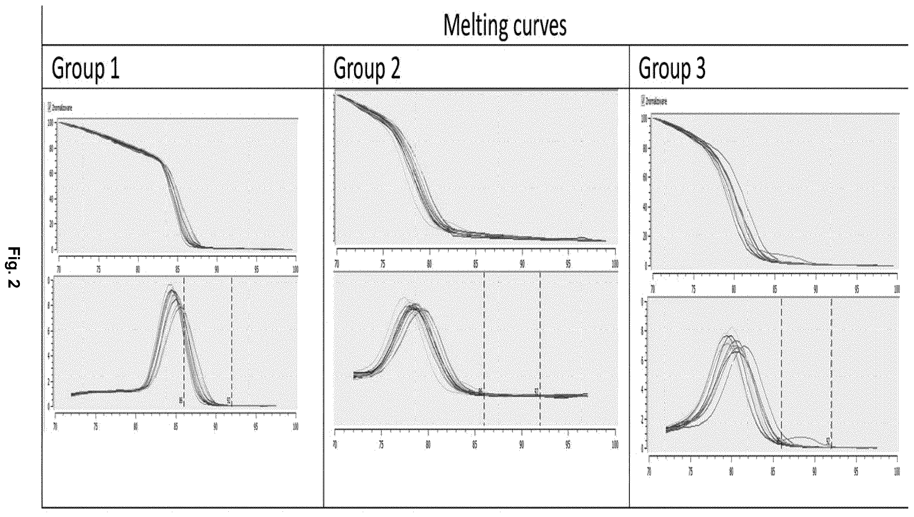

Fig. 2 shows graphs on melting curves in the comparative PCR assay for detection of *Clostridioides difficile* strains of group 1 (R2091 strain of B1/NAP1/027 group), group 2 (ATCC 9689 strain, closely related to B1/NAP1/027 group) and group 3 (630 strain, no NAP1 group), respectively. When comparing the melting curves of the respective groups it is apparent, that for group 1 the melting temperature is 84 °C, which is 4°C higher than the melting temperature for groups 2 and 3.

Combining the results of the growth curves and the melting curves, it is apparent that for group 1 the expected hybridization and subsequent amplification between the inventive primers, in particular inventive primer pair and the chloramphenicol acetyltransferase target sequence took place, whereas for groups 2 and 3 the respective growth and melting curves are associated with the creation of primer-dimers not resulting in the expected amplification of the of the chloramphenicol acetyltransferase target sequence. Table 4 provides an overview of the respective executive examples 1 and 2.

**Table 4:**

| ***C. difficile* strain** | **Group** | **Sample source material** | **Result *in silico*** | **Result of PCR study** |
|---|---|---|---|---|
| R20291 | Group 1 | DNA | + | + |
| ATCC 9689 | Group 2 | Bacteries | - | - |
| 630 | Group 3 | Bacteries | - | - |

Table 4 shows that the results of the PCR study of executive example 2 approves the *in silico* results of executive example 1, which means that the inventive primer pair is highly specific for the detection of hyper-virulent *Clostridioides difficile* strain of the B1/NAP1/027 group in comparison to closely related strains of group 2 or other strains of group 3. The PCR amplification method is also highly sensitive as well as simple, time and cost effective.

## Claims

1. A primer pair for hybridizing to at least a portion of a chloramphenicol acetyltransferase gene coded in a transposon of family CTn-027 of a hyper-virulent *Clostridioides difficile* strain of the B1/NAP1/027 group, **characterized in that** the primer pair comprises or consists of
a. a first isolated oligonucleotide a) comprising or consisting of at least 88 %, 89 %, 90 %, 91 %, 92 %, 93 %, 94 %, 95 %, 96 %, 97 %, 98 %, 99 % or 100 % sequence identity to SEQ ID No. 1, wherein the oligonucleotide comprises at least 10 nucleotides, or an isolated oligonucleotide comprising or consisting of a complimentary oligonucleotide sequence to the isolated oligonucleotide comprising or consisting of at least 88 %, 89 %, 90 %, 91 %, 92 %, 93 %, 94 %, 95 %, 96 %, 97 %, 98 %, 99 % or 100 % sequence identity to SEQ ID No. 1, and
b. a second isolated oligonucleotide b) hybridizing to at least a portion of a chloramphenicol acetyltransferase gene coded in a transposon of family CTn-027 of a hyper-virulent *Clostridioides difficile* strain of the B1/NAP1/027 group, preferably comprising or consisting of at least 90 %, 91 %, 92 %, 93 %, 94 %, 95 %, 96 %, 97 %, 98 %, 99 % or 100 % sequence identity to SEQ ID No. 2, wherein the oligonucleotide comprises at least 10 nucleotides, or an isolated oligonucleotide comprising or consisting of a complimentary oligonucleotide sequence to the oligonucleotide comprising or consisting of at least 90 %, 91 %, 92 %, 93 %, 94 %, 95 %, 96 %, 97 %, 98 %, 99 % or 100 % sequence identity to SEQ ID No. 2.

2. An oligonucleotide probe for hybridizing to at least a portion of a chloramphenicol acetyltransferase gene coded in a transposon of family CTn-027 of a hyper-virulent *Clostridioides difficile* strain of the B1/NAP1/027 group, **characterized in that** the oligonucleotide probe comprises or consists of an isolated oligonucleotide a) comprising or consisting of at least 88 %, 89 %, 90 %, 91 % , 92 % , 93 %, 94 %, 95 %, 96 %, 97 %, 98 %, 99 % or 100 % sequence identity to SEQ ID No. 1, wherein the oligonucleotide comprises at least 10 nucleotides, or an isolated oligonucleotide comprising or consisting of a complimentary oligonucleotide sequence to the isolated oligonucleotide comprising or consisting of at least 88 %, 89 %, 90 %, 91 % , 92 % , 93 %, 94 %, 95 %, 96 %, 97 %, 98 %, 99 % or 100 % sequence identity to SEQ ID No. 1.

3. The primer pair according to claim 1 or the oligonucleotide probe according to claim 2, wherein the oligonucleotide a) and / or the oligonucleotide b) independently from each other comprise 15 or more nucleotides, preferably 20 or more nucleotides.

4. The primer pair according to claim 1 or 3 or the oligonucleotide probe according to claim 2 or 3, wherein the oligonucleotide coding the chloramphenicol acetyltransferase gene in the transposon of family CTn-027 of a hyper-virulent *Clostridioides difficile* strain comprises or consists of SEQ ID No. 3.

5. The primer pair according to any one of claims 1, 3 or 4 or the oligonucleotide probe according to any one of claims 2 to 4, wherein the oligonucleotide coding the chloramphenicol acetyltransferase gene in the transposon of family CTn-027 of a hyper-virulent *Clostridioides difficile* strain comprises or consists of SEQ ID No. 4.

6. The primer pair according to any one of claims 1, and 3 to 5 or the oligonucleotide probe according to any one of claims 2 to 5, wherein the oligonucleotide a) and/or the oligonucleotide b) independently from each other further comprise one or more added functionalities, preferably selected from quenchers and/or fluorophores, radioisotopes and/or one or more modified nucleotides, preferably locked nucleic acid (LNA).

7. An oligonucleotide set for use in a nucleic acid amplification reaction or in a nucleic acid detection reaction for determining the presence of a hyper-virulent *Clostridioides difficile* strain of the B1/NAP1/027 group in a sample, **characterized in that** the oligonucleotide set comprises the primer pair according to any one of claims 1 and 3 to 6 or the oligonucleotide probe according to any one of claims 2 to 6 and one or more additives.

8. A kit for detecting the presence of a hyper-virulent strain of *Clostridioides difficile* of the B1/NAP1/027 group in a sample comprising the primer pair according to any one of claims 1 or 3 to 6, the oligonucleotide probe according to any one of claims 2 to 6, or the oligonucleotide set according to claim 7, **characterized in that** the oligonucleotide a) and oligonucleotide b), if present, hybridize under stringent conditions to at least part of SEQ ID No. 3, preferably at least part of SEQ ID No. 4.

9. The kit for detecting the presence of a hyper-virulent strain *of Clostridioides difficile* of the B1/NAP1/027 group in a sample according to claim 8, wherein the kit further comprises one, two, three, four or more constituents selected from the group of enzymes, such as polymerase, ligase, helicase, or recombinase; deoxynucleotides; control template; buffer and additives.

10. The kit for detecting the presence of a hyper-virulent strain of *Clostridioides difficile* of the B1/NAP1/027 group in a sample according to claim 9, wherein the buffer is dissolved in a solvent, preferably nuclease free water, and comprises or consists of one or more constituents selected from the group of salts, such as potassium chloride (KCI), ammonium sulfate (NH₄)₂SO₄, tris(hydroxymethyl)aminomethane hydrochloride (Tris-HCI), or proteins, such as albumin including Bovine Serum Albumin; and/or the additives comprises or consists of one, two or more constituents selected from the group of co-factors, such as magnesium chloride (MgCl₂); dimethyl sulfoxide (DMSO); fluorophores, such as fluorescent dyes intercalating into double stranded nucleic acid strands; and quenchers.

11. The kit for detecting the presence of a hyper-virulent strain of *Clostridioides difficile* of the B1/NAP1/027 group in a sample according to any one of claims 8 to 10, wherein the stringent condition comprises nuclease free water and 0.05 to 0.1 U/µl polymerase, preferably taq DNA polymerase, 10 to 200 mM Tris-HCI, 0.5 to 5 mM, preferably 3 mM MgCl₂, 50 to 500, preferably 50 mM KCI and a pH in the range of 6.8 to 9.1, preferably pH of 8.4 to 8.8.

12. A method of determining the presence of a hyper-virulent strain of *Clostridioides difficile* of the B1/NAP1/027 group in a sample, the method comprises or consists of:
a. performing a nucleic acid hybridization and amplification reaction comprising a denatured nucleotide extract of the sample as a template and a primer pair according to any one of claims 1 and 3 to 6 or an oligonucleotide set of claim 7 or a kit according to any one of claims 8 to 11 in order to generate an amplicon of at least a portion of a chloramphenicol acetyltransferase gene coded in a transposon of family CTn-027 of a hyper-virulent *Clostridioides difficile* strain of the B1/NAP1/027 group,
b. detecting the amplicon generated in step a), and
c. determining the presence of a hyper-virulent strain of *Clostridioides difficile* of the B1/NAP1/027 group in the sample as a function of the detected amplicon in step b).

13. The method of determining the presence of a hyper-virulent strain of *Clostridioides difficile* of the B1/NAP1/027 group in a sample according to claim 12, wherein the amplification reaction is a polymerase chain reaction (PCR), such as Real-Time PCR (qPCR), a ligase chain reaction (LCR), ligase detection reaction (LDR), multiplex PCR-ligase detection reaction assay, multiplex ligation dependent probe amplification (MLPA), multiplex oligonucleotide ligation PCR (MOL-PCR), Isothermal DNA amplification, Rolling Circle Amplification (RCA), Helicase-Dependent Amplification (HAD), Signal-Mediated Amplification of RNA Technology (SMART), Recombinase Polymerase Amplification (RPA), Polymerase Spiral Reaction (PSR), Strand displacement amplification.

14. The method of determining the presence of a hyper-virulent strain of *Clostridioides difficile* of the B1/NAP1/027 group in a sample according to claim 12 or 13, wherein the amplicon is detected by use of a non-specific detection method, such as detection of fluorescence.

15. The method of determining the presence of a hyper-virulent strain of *Clostridioides difficile* of the B1/NAP1/027 group in a sample according to any one of claims 12 to 14, wherein the sample is a human sample, an animal sample or a food sample, preferably selected from a stool sample, a smear test sample, a body fluid sample, or a tissue sample.
